(19) Europäisches Patentamt
European Patent Office
Office européen des brevets

(11) Publication number: **0 491 041 A1**

(12) # EUROPEAN PATENT APPLICATION published in accordance with Art. 158(3) EPC

(21) Application number: **90913255.7**

(51) Int. Cl.[5]: **A61K 31/445**

(22) Date of filing: **06.09.90**

(86) International application number:
**PCT/JP90/01141**

(87) International publication number:
**WO 91/03242 (21.03.91 91/07)**

(30) Priority: **07.09.89 JP 232135/89**
**10.05.90 JP 120422/90**

(43) Date of publication of application:
**24.06.92 Bulletin 92/26**

(84) Designated Contracting States:
**AT BE CH DE DK ES FR GB IT LI LU NL SE**

(71) Applicant: **NIPPON SHINYAKU COMPANY, LIMITED**
**14, Kisshoin Nishinosho Monguchicho**
**Minami-ku Kyoto-shi Kyoto 601(JP)**

(72) Inventor: **YOSHIKUNI, Yoshiaki**
**1-22, Hatoyama 4-chome**
**Uji-shi, Kyoto 611(JP)**
Inventor: **NAKANE, Masayuki**
**4-45, Marunouchicho**
**Otsu-shi, Shiga 520(JP)**

(74) Representative: **Kügele, Bernhard et al**
**c/o NOVAPAT-CABINET CHEREAU 9, Rue du Valais**
**CH-1202 Genève(CH)**

(54) **ANTIVIRAL DRUG.**

(57) Some of the compounds represented by general formula (I) have been found to have an especially excellent antiviral action including HIV virus inhibition (I), wherein R represents ($\alpha$) or ($\beta$), $R^6$ represents hydrogen or lower alkyl, t is 1 or 2, a symbol

-----

represents either a single bond or a double bond, and $R^{11}$ represents chlorine or fluorine.

EP 0 491 041 A1

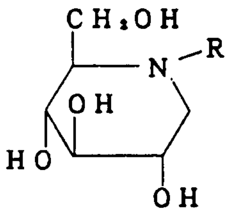
〔I〕

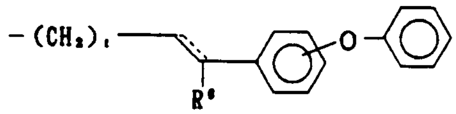
(α)

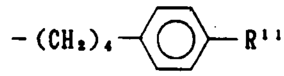
(β)

(Technical Field)

The present invention relates to antiviral drug wherein a compound expressed by the following general formula (I)

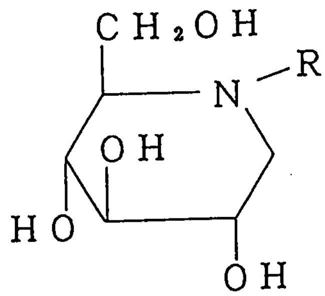

(I)

or pharmalogically-acceptable salt thereof is a main ingredient.

In the formula, R is a substituent selected from a group consisting of the following (1) to (8).

(1) 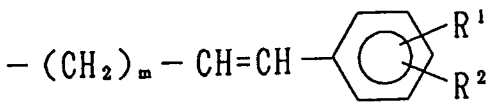

in which $R^1$ is hydrogen, halongen or lower alkyl;
$R^2$ is lower alkyl or lower alkoxy; and m is 1, 2 or 3.

(2) 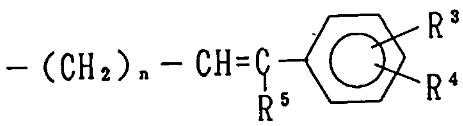

in which $R^3$ and $R^4$ are same or different and are hydrogen, halogen, lower alkyl, lower alkoxy, lower alkoxyalkoxy, lower dialkylamkno or trifluoromethyl; n is 1 or 2; and $R^5$ is lower alkyl or phenyl.

(3) 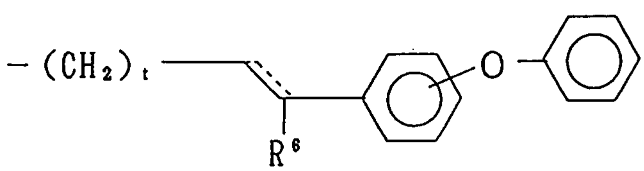

in which $R^6$ is hydrogen or lower alkyl; t is 1 or 2; and



is a single bond or a double bond.

(4) 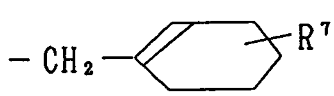

in which $R^7$ is lower alkenyl.

(5) $-CO(CH_2)_q-CH_3$

in which q is an integer of 10 to 20.

(6) 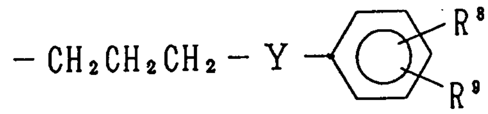

in which $R^8$ and $R^9$ are same or different and are hydrogen, halogen or lower alkyl excluding the case where both $R^8$ and $R^9$ are hydrogens at the same time; and Y is $-CH_2-$ or -O-.

(7) 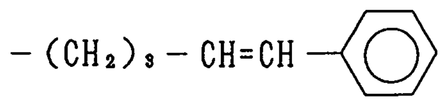

(8) 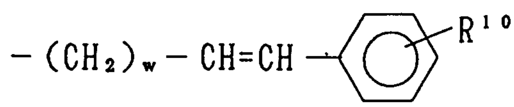

in which $F^{10}$ is halogen; and w is 1 or 2 wherein the substances are limited to the case where there are two R's substituted at N in the compound (I) whereby ammonium ion is formed.

Technical Background

Usually, virus is a fine microorganism which has DNA or RNA as a gene and is composed of that and constituting protein only. There are many common diseases such as influenza, a cold in the nose and herpes which are caused by virus. Little attention has been paid to viral diseases since most of them are light ones. However, with a finding of new diseases with high death rate and of new virus to which vaccine is not so effective, there has been a marked progress in the study on virus.

Many antiviral drugs now being used are antiherpes agents and most of them are derivatives of nucleic acid such as, for example, idoxuridine,
arabinosyladenine, acyclovir, aditothymidine, etc. They can be said to be nucleic acid antagonists having an active point for replication of virus genes.

However, those compounds have disadvantages that their antiviral action is insufficient in terms of activity and that their actual value is remarkably decreased due to serious side effects.

In Japanese Laid Open 01/151593, there is a disclosure that certain types of 2-hydroxymethylene-3,4,5-trihydroxypiperidine derivatives can be used as antiviral drugs. However, even those compounds cannot be expected to exhibit marked effect as antiviral drugs since, in the experimental system of in vitro, $IC_{50}$ is 10 μg/ml.

In Japanese Laid Open 02/131425, there is a disclosure that certain types of N-substituted 1-deoxynojirimycin derivatives exhibit antiviral action. However, even those compounds exhibit nearly the same effect as above and, in addition, their structure is clearly different from that of our invention.

Disclosure of the Invention

During the course of antiviral screening of a lot of compounds, the present inventors have found that the compounds of the present invention exhibit very strong antiviral action and accomplished the present invention. Accordingly, an object of the present invention is to offer antiviral drugs with strong effect.

The characteristic feature of the present invention is that the compound of the present invention

expressed as (I) hereinabove exhibits antiviral action.

The compounds of the present invention have been known by, for example, European Patent Laid Open 0 000 947 and others.

The compounds of the present invention exhibit several to about one hundred times or even stronger antiviral action than the compounds disclosed in Japanese Laid Open 01/151593 which was referred to already.

Best Mode in Embodying the Invention

Structures of the present invention compounds will be given as hereunder.

In (1) among the compounds of the present invention expressed by (I), examples of halogen expressed by $R^1$ are chlorine, fluorine, bromine and iodine. Examples of lower alkyl expressed by $R^1$ and $R^2$ are methyl, ethyl, propyl, butyl, etc. In the case of propyl and butyl, they may be either straight or branched.

Examples of lower alkoxy expressed by $R^2$ are methoxy, ethoxy, propoxy, butoxy, etc. The propyl and butyl in this case may be either straight or branched.

Typical examples of the compounds of the present invention when the substituent is (1) are as follows:

4-[4-(3-Fluoro-4-methylphenyl)-3-butenyl]moranoline (Compound No. 133);
N-[5-(3-Chloro-4-methoxyphenyl)-4-pentenyl]moranoline (Compound No. 148); and
N-[5-(4-Methoxy-2-methylphenyl)-4-pentenyl]moranoline (Compound No. 151).

In (2), examples of the lower alkyl expressed by $R^3$ and $R^4$ are methyl, ethyl, propyl, butyl, etc. In the case of propyl and butyl, they may be straight or branched.

Examples of halogen expressed by $R^3$ and $R^4$ are preferably chlorine though fluorine, bromine and iodine will do as well.

Examples of lower alkoxy expressed by $R^3$ and $R^4$ are methoxy, ethoxy, propoxy, butoxy, etc. and, in that case, propyl and butyl may be either straight or branched.

Examples of lower alkoxyalkoxy expressed by $R^3$ and $R^4$ are methoxymethoxy, methoxyethoxy, methoxypropoxy, methoxybutoxy, ethoxymethoxy, ethoxyethoxy, ethoxypropoxyl, ethoxybutoxy, propoxymethoxy, propoxyethoxy, propoxypropoxy, propoxybutoxy, butoxymethoxy, butoxyethoxy, butoxypropoxy, butoxybutoxy, etc. In that case, propyl and butyl may be either straight or branched.

Examples of lower dialkylamino expressed by $R^3$ and $R^4$ are dimethylamino, diethylamino, di-n-propylamino, diisopropylamino, di-n-butylamino, diisobutylamino, etc. In that case, propyl and butyl may be either straight or branched.

Examples of lower alkyl expressed by $R^5$ are methyl, ethyl, propyl, butyl, etc. In the case of propyl and butyl, they may be either straight or branched.

Typical Examples of the compounds of the present invention wherein the substituent is (2) are as follows:

N-(gamma-Phenyl-4-chlorocinnamyl)moranoline monohydrate (Compound No. 99);
N-[4-(3-Trifluoromethylphenyl)-3-pentenyl]moranoline (Compound No. 113);
N-[4-(2-Methyl-5-isopropylphenyl)-3-pentenyl]moranoline (Compound No. 119);
N-[3-(3-Methoxyethoxyphenyl)-2-butenyl]moranoline (Compound No. 157);
N-(gamma-Methyl-3-ethoxycinnamyl)moranoline (Compound No. 159);
N-[3-(4-Ethoxyphenyl)-2-butenyl]moranoline (Compound No. 160);
N-(gamma-Phenyl-4-fluorocinnamyl)moranoline (Compound No. 100);
N-(gamma-Methyl-3,4-dichlorocinnamyl)moranoline (Compound No. 102);
N-(gamma-Methyl-4-methylcinnamyl)moranoline (Compound No. 104);
N-[4-(2-Methylphenyl)-3-pentenyl]moranoline (Compound No. 109);
N-(gamma-Methyl-4-bromocinnamyl)moranoline (Compound No. 101);
N-(magga-Methyl-4-methoxycinnamyl)moranoline (Compound No. 103);
N-(gamma-Phenyl-4-methoxycinnamyl)moranoline (Compound No. 105);
N-[4-(3-Fluorophenyl)-3-pentenyl]moranoline (Compound No. 107);
N-[4-(4-Fluorophenyl)-3-pentenyl]moranoline (Compound No. 108);
N-[4-(4-Chlorophenyl)-3-pentenyl]moranoline (Compound No. 110);
N-[4-(3-Chlorophenyl)-3-pentenyl]moranoline (Compound No. 111);
N-[4-(2-Chlorophenyl)-3-pentenyl]moranoline (Compound No. 112);
N-[4-(4-Dimethylaminophenyl)-3-pentenyl]moranoline (Compound No. 114);
N-[4-(2-Methoxyphenyl)-3-pentenyl]moranoline (Compound No. 116);
N-[4-(4-Ethoxyphenyl)-3-pentenyl]moranoline (Compound No. 118); and
N-[4-(2-Methyl-5-isopropyl)phenyl-3-pentenyl]moranoline (Compound No. 119).

Examples of lower alkyl expressed as $R^6$ in (3) are methyl, ethyl, propyl, butyl, etc. In the case of propyl and butyl, they may be either straight or branched.

Typical examples of the compounds of the present invention when the substituent is (3) are as follows:

N-[4-(4-Phenoxyphenyl)-3-pentenyl]moranoline (Compound No. 115);

N-[4-(4-Phenoxyphenyl)butyl]moranoline (Compound No. 205);

N-[4-(4-Phenoxyphenyl)-3-butenyl]moranoline (Compound No. 206); and

N-[3-(4-Phenoxyphenyl)-2-propenyl]moranoline (Compound No. 207).

Examples of lower alkenyl expressed as $R^7$ in (4) are n-propenyl, isopropenyl, n-butenyl, isobutenyl, etc.

Typical example of the compounds of the present invention when the substituent is (4) is as follows:

N-[(4-Isopropenyl-1-cyclohexenyl)methyl]moranoline tosylate (Compound No. 106).

Typical examples of the compounds of the present invention when the substituent is (5) are as follows:

N-Stearoylmoranoline (Compound No. 171); and

N-Myristoylmoranoline (Compound No. 174).

Examples of halogen expressed by $R^8$ and $R^9$ in (6) are chlorine, fluorine, bromine, iodine, etc.

Examples of lower alkyl expressed by $R^8$ and $R^9$ are methyl, ethyl, propyl, butyl, etc.

Typical examples of the compounds of the present invention wherein the substituent is (5) are as follows:

N-[3-(4-Chlorophenoxy)propyl]moranoline (Compound No. 93);

N-[4-(4-Chlorophenoxy)butyl]moranoline (Compound No. 141);

N-[4-(3-Fluoro-4-methylphenyl)butyl]moranoline (Compound No. 150); and

N-[4-(4-Fluorophenyl)butyl]moranoline (Compound No. 204).

An example of the compounds of the present invention wherein the substituent is (7) is as follows:

N-(5-Phenyl-4-pentenyl)moranoline tosylate (Compound No. 89).

Examples of halogen expressed by $R^{10}$ in (8) are chlorine, fluorine, bromine, iodine, etc. In the case of (8), only the case wherein there are two R's attaching to N in the compound (I) whereby ammonium ion is constructed is included in the compounds of the present invention.

Typical examples of the compounds of the present invention in such a case are as follows:

N,N-Bis(2-chlorocinnamyl)moranoline ammonium chloride (Compound No. 123); and

N,N-Bis(4-chlorocinnamoyl)moranoline ammonium chloride (Compound No. 201).

Among the above, those which are preferred as antiviral drugs in terms of the ratio of antiviral effect to cycotoxicity are the Compounds of Nos. 89, 121, 133, 115, 93 and 141 and, among those, Nos. 115 and 141 are particularly preferred.

When the compounds are jointly used with antiviral drugs with different action mechanism, the result is advantageous in terms of safety and it is also possible to expect synergistic effect as antiviral drugs.

The compounds of the present invention are basic and, therefore, they can form salts with various acids. When such salts are pharmacologically acceptable, they are of course included in the present invention.

When the compound of the present invention is given as medicine, it is administered to animals including human being as it is or as a pharmaceutical composition containing 0.1-99.5% or, preferably 0.5-90%, of the compound in pharmacuetically-acceptable, nontoxic and inert carrier.

One or more solid, semisolid or liquid diluents, fillers and other auxiliary agents for prescription may be used as carrier. It is desired to give the pharmaceutical composition in a form of unit dosage form. The pharmaceutical composition of the present invention may be administered per os, into tissues, via local part (e.g. via skin) or via rectum. Needless to say, prescription form suitable for each administration route is adopted. For example, oral administration is particularly preferred.

Dose as antiviral drug is preferably decided by taking the state of the patient (e.g. age and body weight), administering route and nature/degree of the disease into consideration but, usually, the amount of the effective ingredient of the present inventjion is within a range of 100 mg to 10 g/day for person and, preferably, 500 mg to 3 g/day for person is most common. In some cases, the dose may be less than the above while, in other cases, it may be more than that. It is preferred to give dividedly - one to three times a day.

Oral administration may be carried out by the pharmaceutical form of solid or liquid dosage unit such as powder, diluted powder, tablets, sugar-coated agents, capsules, granules, suspensions, liquid, sirup, drops, sublingual agents, etc.

Power can be manufactured by making the active substance into a suitable fine size. Diluted powder can be manufactured by making the active substance into suitable fine size followed by mixing with similarly powdered pharmaceutical carrier such as edible carbohydrate (e.g. starch and mannitol) or others.

If necessary, seasoning agent, preservative, dispersing agent, coloring agent, fragrance and others may be mixed therewith.

Capsules can be manufactured by filling the powder prepared as above or granulated one (mentioned in the item of tablets) into capsules such as gelatin capsules. It is also possible that lubricant and fludizing agent such as colloidal silica, talc, magnesium stearate, calcium stearate, solid polyethylene glycol and the like are mixed with the powder followed by subjecting to a filling operation. When disintegrating agent and solubilizing agent such as carboxymethylcellulose, calcium carboxymethylcellulose, lowly substituted hydroxypropylcellulose, calcium carbonate, sodium carbonate, etc. are added, effectiveness of the medicine when the capsules are taken can be improved.

Alternatively, the fine powder of the compound is suspended/dispersed in plant oil, polyethyleneglycol, glycerol and surface active agent followed by packing with gelatin sheet to prepare soft capsules. Tablets can be manufactured by preparing powder mixture, making into granules or slugs, adding lubricant or disintegrating agent thereto and making into tablets by compression. The powder mixture can be manufactured by mixing the suitably pulverized substance with the above-given diluent or a base and mixed, if necessary, with binders (e.g. carboxymethylcellulose sodium, alginates, gelatin, polyvinylpyrrolidone, polyvinyl alcohol, etc.), retardants for dissolution (e.g. paraffin), reabsorbers (e.g. quaternary salts) and adsorbents (e.g. bentonite, kaolin and dicalcium phosphate). The mixed powder is first wetted with a binder (such as sirup, starch paste, gum arabicum, cellulose solution or polymer solution) and then compulsorily passed through a sieve to prepare granules. Instead of making the powder into granules as such, it is also possible that the powder is subjected to a tableting machine and the resulting slugs of incomplete shape are pulverized whereupon granules are resulted.

Granules prepared as such may be mixed with lubricant such as stearic acid, stearates, talc, mineral oil or the like so that sticking of granules can be prevented. Such a lubricated mixture is then made into tablets.

Alternatively, the compounds may be mixed with fluid inert carrier and directly made into tablets without the steps of preparing granules or slugs. Transparent or semi-transparent protective coating comprising closed shellac coating, coating of sugar or polymers and polishing coating comprising wax may be used as well.

Other dosage forms for oral administration such as solution, sirup and elixier may be made into unit dose form wherein its certain amount contains certain amount of the drug. Sirup may be manufactured by dissolving the compound into a suitable aqueous solution with good taste while elixier may be manufactured using nontoxic alcoholic carrier. Suspension may be manufactured by dispersing the compound into nontoxic carrier. If necessary, solubilizing agent and emulsifiers (e.g. ethoxylated isostearyl alcohols, polyoxyethylene sorbitol esters, etc.), preservatives, seasoning agents (e.g. peppermint oil and saccharine) and others may be added thereto.

If necessary, the unit dose form for oral administration may be in a form of microcapsules. Moreover, said prescription may be coated or embedded in polymer or wax so that acting time can be elongated or sustained released effect can be resulted.

Parenteral administration may be carried out using liquid unit dose form for hypodermic, intramuscular or intravenous injection (e.g. in a form of solution or suspension). Those can be manufactured by suspending or dissolving certain amount of the compound into nontoxic liquid carrier (e.g. aqueous or oily medium) meeting with an object of the injection and then said suspension or solution is sterilized. Alternatively, certain amount of the compound is placed in vials and then said vials are sterized with the content followed by sealing tightly. In order to dissolve or mix immediately before administration, preliminary vials or carrier may be prepared in addition to the powdered or freeze-dried effective compound. In order to make the injection solution isotonic, nontoxic salt or solution solution may be added thereto. Moreover, stabilizers, preservatives, emulsifiers, etc. may be jointly used.

Rectal administration can be carried out using suppositories prepared by mixing the compound with low-melting and water-soluble or water-insoluble solid such as polyethyleneglycol, cacao mass, higher esters (e.g. myristyl palmitate) or a mixture thereof.

Antiviral action of the compounds of the present invention can be confirmed as follows.

Antiviral Action Test

(1) Culture of Virus and Preparation of Virus Liquid.

HeLa cells were subjected to subcultured by monolayer culture (Eagle's MEM-10% CS) and infected as M.O.I. (multiplicity of infection) = 0.01.

On the first day, HeLa cells were spread in a 75 $cm^3$ flask and cultured on a one-day growth medium (Eagle's MEM-10% CS). On the second day, old medium was removed after confirming the formation of cell sheet, then M.O.I was made 0.01 PFU/cell and 0.5 ml of virus solution was added. The original virus solution was diluted with PBS-3% FCS solution. Operation of the virus inoculation was conducted by tilting for 1 hour at room temperature. After virus was inoculated on the cells, the virus-inoculated solution was removed, washed twice with 10 ml PBS and the residual virus solution was removed. Then 15 ml of maintenance medium (Eagle's MEM-1% CS) was added and cultured at 37 °C for 2 days. The cells were observed from time to time and the culture was continued until cell fusion and cell rounding were confirmed. After the culture, medium was recovered which was used as a virus solution and was stored at -20 °C until actual use.

(2) Measurement of infection titre of prepared virus (Plaque Assay).

1. Preparation of Cell Sheet.

The 78 $cm^3$ flask containing HeLa cells which were subjected to a monolayer maintenance subculture was taken out, the old medium was discarded, 2-3 ml of 0.25% trypsine (Difco = 1:250)-0.02% EDTA/PBS(-) solution was added, tilted for a few seconds, the trypsine solution was removed and warmed at 37 °C for 5 minutes. Then fresh medium was added, pipetted with a Komagome pipette and the cells were homogeneously dispersed.

Cells were counted using a cytometer, then fresh medium was added, diluted to make the cell concentration 5 x $10^5$ cells/ml, 2 ml of that was added to a laboratory dish of 35 mm diameter and cultured in a $CO_2$-incubator at 37 °C for one day to prepare cell sheet.

2. Virus Infection.

The virus solution was diluted by a diluting solution to make diluted solutions of ten-times difference or step. The laboratory dish wherein HeLa cells were cultured on the previous day was taken out from the incubator, old medium was discarded and the cells were washed with each 2 ml of PBS twice. Properly diluted virus solution (0.2 ml) was added to each laboratory dish, subjected to a tilting at room temperature for 60 minutes immediately and virus was inoculated. Then the virus solution was removed, each 2 ml of 0.5% methylcellulose/Eagle's MEM-1% CS was added and cultured in a $CO_2$ incubator for 4 days.

3. Plaque Assay.

The cultured laboratory dish was taken out from the $CO_2$-incubator and methylcellulose medium was removed. Then cells were washed with 2 ml of PDS, 2 ml of methanol was added, allowed to stand for 10 minutes and cells were immobilized and, at the same time, virus was sterilized. Hematoxylin dyeing solution (2 ml) was added to the laboratory dish which was immobilized with methanol, allowed to stand for about 10 minutes, washed with water and air-dried at room temperature. Since the cells destructed with virus were not dyed, plaque area was noted as a void and the plaque numbers were counted whereby virus value (PFU/ml) (Plaque forming units/ml) was measured.

(3) Screening of Antiviral Drugs.

1. Preparation of Cell Sheet.

HeLa cells during the subculture were dispersed with trypsine liquid-EDTA solution, 5 x $10^5$ cells/pore were embedded on a plate having 24 pores and cultured overnight at 37 °C in a $CO_2$-incubator to prepare cell sheet.

2. Inoculation of Virus.

As mentioned above, old medium was discarded, the cells were washed with PBS, the virus solution was diluted to make M.O.I = 0.002, 0.1 ml of it was added to each pore, subjected to a tilting at room temperature for 60 minutes and virus was inoculated to the cell.

3. Addition of Drug.

Virus solution was removed from the cells after inoculation of virus, the cells were washed with 2 ml of PBS, each 1 ml of maintenance medium was added to each pore, then each 10 µl of the drug was added and the cells were cultured in a $CO_2$-incubator. Culture was conducted for three days.

4. Observation and Judgement.

The cells on the plate were observed under an invert microscope. Cell degeneration effect (CPE) was observed as cell fusion and making the cells round.

The cell degenertion effect by virus observed in the cells to which no drug was added was defined as the state of 100% and the degree of CPE of the cells to which the drug was added was expressed by %. Out of the relation between the amount of the added drug ( µg/ml) and the above %, the amount whereby the CPE became 50% was measuredand this was adopted as MIC (the amount whereby 50% effect can be resulted). The result is given as hereunder:

| Compd.No. | MIC | Compd.No. | MIC |
|---|---|---|---|
| A | >100 | 113 | 30 |
| B | >100 | 114 | 30 |
| 89 | 10 | 115 | 1 |
| 93 | 30 | 116 | 30 |
| 99 | 30 | 118 | 30 |
| 100 | 10 | 119 | 3 |
| 101 | 30 | 123 | 3 |
| 102 | 10 | 133 | 30 |
| 103 | 30 | 141 | 1 |
| 104 | 10 | 148 | 30 |
| 105 | 100 | 150 | 30 |
| 106 | 30 | 151 | 30 |
| 107 | 30 | 157 | 100 |
| 108 | 30 | 159 | 30 |
| 109 | 30 | 160 | 10 |
| 110 | 10 | 171 | 30 |
| 111 | 30 | 174 | 10 |
| 112 | 30 | | |

In the above table, A and B stand for N-n-butylmoranoline and N-ethylmoranoline, respectively. It is clear that, as compared with those two, the compounds of the present invention exhibited several tens to one hundred times higher CPE-inhibiting effect.

Anti-HIV Action

(1) Materials.

1. Virus.

The virus used was HIV-1 (Human Immunodeficiency Virus, Type 1, Strain IIIB).

2. Cells.

Molt-4 cells were used for preparing the seed virus while, for the measurement of virus infection titre and screening of the drug, MT-4 cells were used. (Molt-4 Cells: CD4 Positive Human T Cell Line; MT-4 Cells: T-Lymphotropic Virus Type 1-Positive T Cell Line).

3. Measurement of Virus Infection Titre.

Virus titre was measured by a plaque assay according to a method by Yamamoto et al (Shinji Harada, Yoshio Koyanagi and Naoki Yamamoto: Infectionof HTLV-III/LAV in HTLV-I - Carring Cells MT-2 and MT-4 and Application in a Plaque Assay, Science, 1985, vol.229, pages 563-566).

4. Culture of the Cells.

Molt-4 cells and MT-4 cells were subjected to subculture for every three days using a medium prepared by adding 10% FBS (Fetal Bovine Serum) to RPMI-1640 medium and, under the atmosphere of 5% $CO_2$/95% air, culture was conducted at 37° C.

5. Preparation of Seed Virus Solution.

Molt-4 cells were infected with HIV-1 at the multiplicity of infection (M.O.I.) of 0.01 PFU (Plaque Forming Unit)/cell followed by culturing for 6 to 8 days. The culture liquid was recogered by a slow-speed centrifugation and the supernatant liquid (wherefrom cells were removed) was stored at -20° C until actual use as a original virus solution. Incidentally the virus titre of the culture liquid was measured by a plaque assay method.

(2) Methods.

1. Preparation of the Cells.

MT-4 cells were recovered by a slow-speed centrifugation and washed three times with PBS (phosphate buffered saline) solution. Then the cells were further washed twice with RPMI-1640 medium containing no serum, suspended on the same medium and the cell concentration was adjusted to 1 x $10^6$ cells/ml. The cell concentration was adjusted by taking a part of the cell suspension, adding equivalent amount of 0.3% trypan blue/PBS solution thereto and counting the living cell numbers. The cell suspension was placed in dishes (Falcon Primaria) of 3.5 cm diameter so that meach dish contained 2 ml of the suspension. They were allowed to stand for 1-2 hours under the atmosphere of 5% $CO_2$/95% air at 37° C. When the cells were fixed on the bottom of the dish (being confirmed by an invert microscope), virus was inoculated.

2. Inoculation of Virus.

Original virus solution was diluted with 3% FBS/PBS to make M.O.I. 0.01, 0.2 ml of it was added to cell sheet (wherefrom the medium was removed) at the amount of 0.2 ml/dish and infection was conducted. Adsorption of the virus was conducted at room temperature for 1 hour.

3. Addition of the Drug and the After-Treatment.

Unadsobred virus solution was removed from the cell sheet and 2 ml of medium (prepared by adding 5% FBE to RPMI-1640) was added to each dish. Then a solution or suspendion of the drug in 50% ethanol was added in an amount of 1/100 volume to the culture liquid, mixed and immediately subjected to a stand culture at 37° C for 60-65 hours under the atmosphere of 5%$CO_2$/95% air. After that, the dish wherefrom the medium was removed was dipped in methanol solution for 15 minutes or longer to immobilize the cells. After the methanol immobilization, production of virus antigen (p24) in the cell was observed by an indirect fluorescent antibody method.

4. Indirect Fluorescent Antibody Method.

After being immobilized with methanol, mouse monoclonal antibody (IgG: Chemicon Co MAB-880A) to HIV-1 p24 nucleocapside protein was diluted 300 times, each 200 µl of it was added to each dish, allowed to stand at room temperature for 1 hour and the primary antibody reaction was conducted. Then the primary antibody solution was removed from each dish and washed with PBS solution four times every 15 seconds then another four times every 15 minutes. Then each 200 µl of 100 times diluted solution of FITC antimouse IgG antibody (Goas: Cappel) was added to each dish and allowed to stand at room temperature for 1 hour to carry out the secondary antibody reaction. Washing of this was conducted by the same manner as in the primary antibody reaction. Dilution of the antibody was conducted by PBS/0.03% sodium azide solution. Finally 50% glycerol/PBS solution was dropped into each dish, a cover glass (Matsunami; 22 mm diameter) was applied thereon and the surrounding part was sealed with Semedyne (an acetone solution). Production of virus antigen in the cells was observed by a fluorescent microscope and the effect of the drug was judged.

5. Evaluation of the Anti-HIV Activity of the Drug.

Central column region of the infected cell samples under the covered glass was observed and the numbers of the focuses (cell groups wherein five or more adjacent cells were fluorescened were defined as "focus") of the cells which were positive to fluorescence by anti-p24 antibody were counted. Judgement of the drug effect was conducted by calculating the ratio of the focus numbers of the samples treated with the drug to those of the control samples and the concentration of the drug ($IC_{50}$) showing 50% inhibition for focus formation was calculated.

(3) Results.

The result is given in the following table.

| Drugs Used (Compound Numbers) | Anti-HIV Activity IC$_{50}$ ($\mu$g/ml) |
|---|---|
| Moranoline | 40.8 |
| 89 | 2.4 |
| 93 | 2.4 |
| 99 | 11.5 |
| 100 | 7 |
| 101 | 4.6 |
| 102 | 2 |
| 103 | 5.6 |
| 104 | 5.6 |
| 106 | 27 |
| 107 | 11 |
| 108 | 1.4 |
| 109 | 3.4 |
| 110 | 4 |
| 111 | 4.6 |
| 112 | 4 |
| 113 | 6.4 |
| 114 | 1.5 |
| 115 | 0.1 |
| 116 | 2.4 |
| 118 | 3.5 |
| 119 | 0.4 |
| 133 | 1.6 |
| 141 | 0.3 |
| 148 | 3 |
| 150 | 4 |
| 159 | 2.7 |
| 160 | 4.4 |
| 171 | 4.6 |
| 174 | 2.1 |
| 201 | 1.1 |
| 204 | 0.84 |
| 205 | 1.2 |
| 206 | 3.2 |
| 207 | 2.5 |
| A | 30.7 |

It is clear from the above that the compounds of the present invention exhibited excellent effect.

Acute Toxicity Test

Four male mice (ddY strain; six weeks age) were used for each drug tested.

Method of Experiment.

In the intraperitoneal administration, each drug was suspended in 0.5% CMC-physiological saline solution and 0.1 ml (per 10 g body weight of mouse) of the suspension was intraperitoneally given to mouse. In the case of oral administration, each drug was suspended in 0.5% CMC-physiological saline solution and 0.2 ml (per 10 g body weight of mouse) of the suspension was given orally. Observation was started soon after administration and, after observation for one week, the mice were killed by chloroform and subjected to autopsied. $LD_{50}$ of each drug tested was given in the following tables wherefrom it is clear that the compounds of the present invention are safe.

| Intraperitoneal Administration: | |
|---|---|
| Compd.No. | $LD_{50}$ (mg/kg) |
| 103 | > 300 |
| 104 | > 300 |
| 115 | > 300 |
| 141 | > 300 |
| 205 | > 300 |

| Oral Administration: | |
|---|---|
| Compd.No. | $LD_{50}$ (mg/kg) |
| 103 | > 5,000 |
| 104 | > 5,000 |
| 115 | > 5,000 |
| 141 | > 5,000 |
| 205 | > 5,000 |

Now the present invention will be further illustrated by way of the examples of the formulations of the compounds of the present invention.

Example 1.

To the compound of the present invention (Compd.No.115) were added the following substances per tablet followed by preparing the tablet by conventional means.

In one tablet (300 mg):

| | |
|---|---|
| Compound of the Invention (No.115) | 200 mg |
| Lactose | 50 mg |
| Corn starch | 20 mg |
| Lowly-substituted hydroxypropylcellullose | 15 mg |
| Hydroxypropylcellulose | 5 mg |
| Magnesium stearate | 10 mg |
| **Total** | 300 mg |

Example 2.

To the compound of the present invention (Compd.No.141) were added the folllowing substances per table followed by preparing the table by conventional means.

In one tablet (300 mg):

| | |
|---|---|
| Compound of the Invention (No.141) | 200 mg |
| Lactose | 50 mg |
| Corn starch | 20 mg |
| Lowly-substituted hydroxypropylcellulose | 15 mg |
| Hydroxypropylcellulose | 5 mg |
| Magnesium stearate | 10 mg |
| **Total** | 300 mg |

Example 3.

To the compound of the present invention (Compd.No.205) were added the following substances per tablet followed by preparing the tablet by conventional means.

In one tablet (300 mg):

| | |
|---|---|
| Compound of the Invention (No.205) | 200 mg |
| Lactose | 50 mg |
| Corn starch | 20 mg |
| Lowly-substituted hydroxypropycellulose | 15 mg |
| Hydroxypropylcellulose | 5 mg |
| Magnesium stearate | 10 mg |
| **Total** | 300 mg |

Example 4.

To the compound of the present invention (Compd.No.115) were added the following substances per ampoule followed by preparing the injection solution by conventional means.

In one ampoule (10 ml):

| | |
|---|---|
| Compound of the Invention (No. 115) | 200 mg |
| Sodium chloride | 90 mg |
| Distilled water for injection | q.s. |
| **Total** | 10 ml |

Example 5.

To the compound of the present invention (Compd.No.141) were added the following substances per ampule followed by preparing the injection solution by conventional means.

In one ampoule (10 ml):

| | |
|---|---|
| Compound of the Invention (No. 141) | 200 mg |
| Sodium chloride | 90 mg |
| Distilled water for injection | q.s. |
| **Total** | 10 ml |

Example 6.

To the compound of the present invention (Compd. No. 205) were added the following substances per ampoule followed by preparing the injection solution by conventional means.

In one ampoule (10 ml):

| | |
|---|---|
| Compound of the Invention (No. 205) | 200 mg |
| Sodium chloride | 90 mg |
| Distilled water for injection | q.s. |
| **Total** | 10 ml |

## Claims

**1.** Antiviral drug containing the compound expressed by the following general formula (I) or pharmacologically-acceptable salt thereof as a main ingredient.

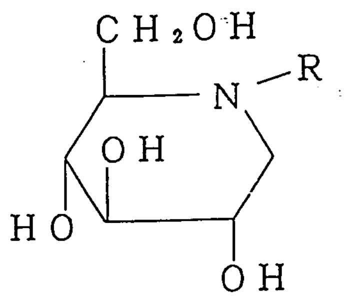

(I)

wherein R is a substituent selected from a group consisting of the following (1) to (8).

(1)

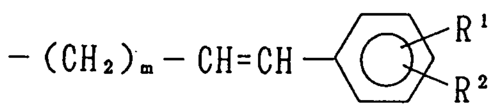

in which $R^1$ is hydrogen, halogen or lower alkyl; $R^2$ is lower alkyl or lower alkoxy; and m is 1, 2 or 3.

(2)

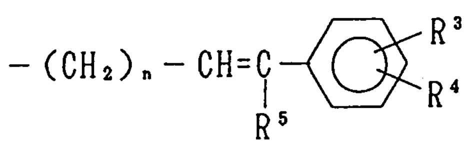

in which $R^3$ and $R^4$ are same or different and are hydrogen, halogen, lower alkyl, lower alkoxy, lower alkoxyalkoxy, lower dialkylamino or trifluoromethyl; n is 1 or 2; and $R^5$ is lower alkyl or phenyl.

(3)

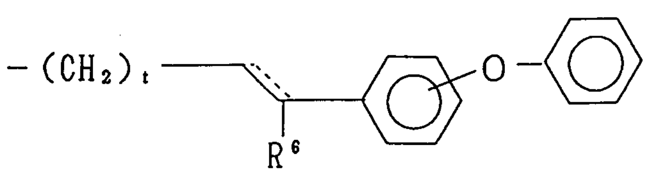

in which $R^6$ is hydrogen or lower alkyl; t is 1 or 2; and

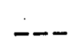

is a single or a double bond.

(4)

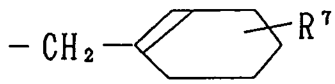

in which $R^7$ is lower alkenyl.

(5) $-CO(CH_2)_q-CH_3$

in which q is an integer of 10 to 20.

(6)

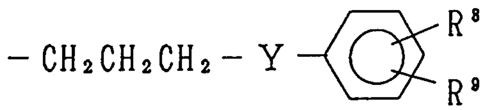

in which $R^8$ and $R^9$ are same or different and are hydrogen, halogen or lower alkyl excluding the case where both $R^8$ and $R^9$ are hydrogen at the same time; and Y is $-CH_2-$ or -O-.

(7)

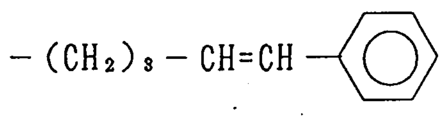

(8)

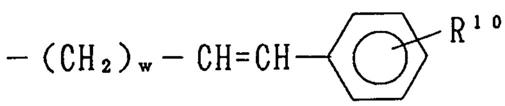

in which $R^{10}$ is halogen and w is 1 or 2 and, in this case, limitation is made to the case only when there are two R's substituting at N in the compound (I) whereby ammonium ion is constructed.

**2.** Antiviral drug of claim 1 wherein the substituent R is the following

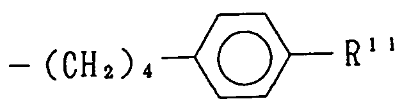

in which $R^{11}$ is chlorine or fluorine.

# INTERNATIONAL SEARCH REPORT

International Application No PCT/JP90/01141

**I. CLASSIFICATION OF SUBJECT MATTER** (if several classification symbols apply, indicate all) [6]

According to International Patent Classification (IPC) or to both National Classification and IPC

Int. $Cl^5$ A61K31/445

**II. FIELDS SEARCHED**

Minimum Documentation Searched [7]

| Classification System | Classification Symbols |
|---|---|
| IPC | A61K31/445, C07D211/44, 211/46 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| X | EP, A, 315017 (Bayer A. G.), 10 May 1989 (10. 05. 89) & JP, A, 1-151593 | 1, 2 |
| P | EP, A, 350012 (Meiji Seika Kaisha, Ltd.), 21 May 1990 (21. 05. 90) & JP, A, 2-131425 | 1, 2 |
| A | EP, A, 34784 (Bayer A. G.), 2 September 1981 (02. 09. 81) & JP, A, 56-133267 | 1 |
| A | JP, A, 56-103163 (Nippon Shinyaku Co., Ltd.), 18 August 1981 (18. 08. 81) | 1 |
| A | JP, A, 56-83409 (Nippon Shinyaku Co., Ltd.), 8 July 1981 (08. 07. 81) | 1 |
| A | JP, A, 55-47655 (Nippon Shinyaku Co., Ltd ), 4 April 1980 (04. 04. 80) | 1, 2 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| November 16, 1990 (16. 11. 90) | December 3, 1990 (03. 12. 90) |
| **International Searching Authority** | **Signature of Authorized Officer** |
| Japanese Patent Office | |

**FURTHER INFORMATION CONTINUED FROM THE SECOND SHEET**

| | | |
|---|---|---|
| | & US, A, 4,533,668 | |
| A | JP, A, 55-98163 (Nippon Shinyaku Co., Ltd.), 25 July 1980 (25. 07. 80) & GB, A, 2020278 | 1 |
| A | EP, A, 328111 (Meiji Seika Kaisha, Ltd.), 16 August 1989 (16. 08. 89) | 1, 2 |

V.☐ **OBSERVATIONS WHERE CERTAIN CLAIMS WERE FOUND UNSEARCHABLE** [1]

This international search report has not been established in respect of certain claims under Article 17(2) (a) for the following reasons:

1.☐ Claim numbers , because they relate to subject matter not required to be searched by this Authority, namely:

2.☐ Claim numbers , because they relate to parts of the international application that do not comply with the prescribed requirements to such an extent that no meaningful international search can be carried out, specifically:

3.☐ Claim numbers , because they are dependent claims and are not drafted in accordance with the second and third sentences of PCT Rule 6.4(a).

VI.☐ **OBSERVATIONS WHERE UNITY OF INVENTION IS LACKING** [2]

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.